# EUROPEAN PATENT APPLICATION

(11) **EP 3 061 358 A1**
(43) Date of publication of application: **31.08.2016**
(21) Application number: 15156746.8
(22) Date of filing: 26.02.2015
(51) Int. Cl.: A24F 47/00

(54) **Electronic smoking device with an air pre-heating element**

(71) Applicant: Fontem Holdings 2 B.V., 1083 HN Amsterdam (NL)
(72) Inventor: Borkovec, Vaclav, 22761 Hamburg (DE); Biel, Stefan, 22761 Hamburg (DE)
(74) Representative: Gulde & Partner

(57) **Abstract**

A cartomizer adapted for an electronic smoking device (10), comprises an elongated, hollow body (14) having a mouthpiece opening (36) in a first end and a coupling adapted to couple to a main body (12) of an electronic smoking device (10) in an opposite second end, an atomizer (26) operable to create an aerosol for inhalation by a user through the mouthpiece opening (36) by atomizing a liquid supplied from a liquid store (34) and arranged inside the body (14), wherein an air flow path (40) extends from an air inlet (38) to the atomizer (26) and an air preheating element (42) arranged in the flow path (40) upstream of the atomizer (26).

## Description

### FIELD OF INVENTION

The present invention relates generally to electronic smoking devices and in particular electronic cigarettes.

### BACKGROUND OF THE INVENTION

An electronic smoking device, such as an electronic cigarette (e-cigarette), usually has a housing accommodating an electric power source (e.g. a single use battery or a rechargeable battery), and an electrically operable atomizer. The atomizer vaporizes or atomizes liquid supplied from a reservoir and provides vaporized or atomized liquid as an aerosol. Control electronics control the activation of the atomizer. In many electronic cigarettes, an airflow sensor is provided within the electronic smoking device which detects a user puffing on the device (e.g., by sensing an under-pressure or an air flow pattern through the device). The airflow sensor indicates or signals the puff to the control electronics. Alternatively, a button may be used to switch on the electronic smoking device to generate a puff of flavour. When a puff is detected, the control electronics supplies electrical power to the atomizer thereby creating vaporized liquid as an aerosol.

The performance of electronic smoking devices may vary due to changing ambient temperatures.

### SUMMARY OF THE INVENTION

In accordance with one aspect of the present invention there is provided a cartomizer adapted for an electronic smoking device including an elongated, hollow body having a mouthpiece opening in a first end and a coupling adapted to couple to a main body of an electronic smoking device in an opposite second end. The cartomizer further includes an atomizer operable to create an aerosol for inhalation by a user through the mouthpiece opening by atomizing a liquid supplied from a liquid store and arranged inside the body. An air flow path extends from an air inlet to the atomizer. An air pre-heating element is arranged in the flow path upstream of the atomizer.

The characteristics, features and advantages of this invention and the manner in which they are obtained as described above, will become more apparent and be more clearly understood in connection with the following description of exemplary embodiments, which are explained with reference to the accompanying drawings.

### BRIEF DESCRIPTION OF THE DRAWINGS

In the drawings, same element numbers indicate same elements in each of the views:
Figure 1 is a schematic cross-sectional illustration of an exemplary e-cigarette;
Figure 2 is a schematic illustration of an exemplary e-cigarette with a first embodiment of an air pre-heating element;
Figure 3 is a schematic illustration of an exemplary e-cigarette with a second embodiment of an air pre-heating element;
Figure 4 is a schematic illustration of an exemplary e-cigarette with a third embodiment of an air pre-heating element;
Figure 5 is a schematic illustration of a further exemplary e-cigarette with an air pre-heating element; and
Figure 6 is a schematic wiring diagram of an exemplary e-cigarette with an air pre-heating element.

### DESCRIPTION OF THE PREFERRED EMBODIMENTS

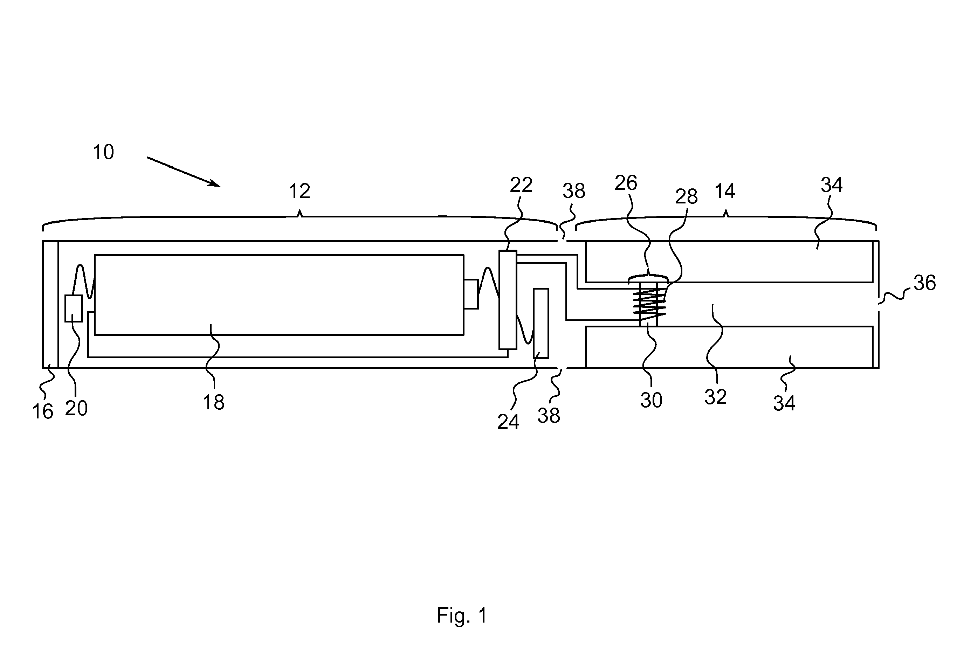

Prior to describing an embodiment of the present invention, the basic structure of an e-cigarette will first be described with reference to Figure 1 which is a schematic cross-sectional illustration of an exemplary e-cigarette.

As is shown in Figure 1, an e-cigarette 10 ordinarily comprises a cylindrical housing having a main body 12 and a mouthpiece portion 14. Together the main body 12 and the mouthpiece portion 14 form a cylindrical tube which is approximately the same size and shape as a conventional cigarette. The main body 12 and mouthpiece 14 are typically made of steel or hardwearing plastic and act to provide a housing to contain the operative elements of the e-cigarette 10. The main body 12 and a mouthpiece portion 14 may be configured to fit together by means of a friction push fit. Alternatively in some embodiments a screw fit may be provided enabling the main body 12 and mouthpiece portion 14 to be attached to one another. Alternatively in some e-cigarettes the main body 12 and mouthpiece portion 14 may be parts of a single integrally formed tube.

An end cap 16 is provided at the end of the main body 12 remote from the mouthpiece portion 14 enclosing that end of the main body 12. The end cap 16 is typically made from translucent plastic.

A battery 18 is provided within the central cavity enclosed by the main body 12. Also contained within the central cavity defined by the main body 12 are a light emitting diode (LED) 20, control electronics 22 and an airflow sensor 24. The battery 18 is electrically connected to the LED 20 and the control electronics 22 and the airflow sensor 24 is connected to the control electronics 22. In this example the LED 20 is provided at one end of the main body 12, adjacent to the end cap 16 and the control electronics 22 and airflow sensor 24 are provided in the central cavity at the other end of the battery 18 adjacent the mouth piece portion 14.

The airflow sensor 24 acts as a puff detector, detecting a user puffing or sucking on the mouthpiece portion 14 of the e-cigarette 10. The airflow sensor 24 can be any suitable sensor for detecting changes in airflow or air pressure such a microphone switch including a deformable membrane which is caused to move by variations in air pressure.

The control electronics 22 are also connected to an atomizer 26 which in this illustrative example comprises a heating coil 28 which is wrapped around a wick 30 which extends across a central passage 32 provided in the mouthpiece portion 14 of the e-cigarette 10. The dimensions of the central passage 32, the wick 30 and the heating coil 28 are such that the wick 30 and heating coil 28 do not completely block the central passage 32 but rather an air gap is provided either side of the heating coil 28 enabling air to flow past the heating coil 28 and wick 30.

The central passage 32 is surrounded by a cylindrical liquid store 34 with the ends of the wick 30 abutting or extending into the liquid store 34. The wick 30 comprises a porous material such as a bundle of fiberglass fibers such that liquid present in the liquid store 34 is drawn by capillary action from the ends of the wick 30 towards the central portion of the wick 30 encircled by the heating coil 28.

In some embodiments the liquid store 34 will comprise wadding soaked in liquid which encircles the central passage 32 with the ends of the wick 30 abutting the wadding. In other embodiments the liquid store 34 may comprise a toroidal cavity arranged to be filled with liquid to be vaporized with the toroidal cavity enclosed by walls and with the ends of the wick 30 extending into the toroidal cavity.

An air inhalation port 36 is provided at the end of the mouthpiece portion 14 remote from main body 12 of the e-cigarette 10 and a pair of air inlets 38 are provided in the housing at the intersection between the main body 12 and the mouthpiece portion 14 adjacent the airflow sensor 24 with the central passage 32 within the mouthpiece portion 14 of the e-cigarette 10 extending from adjacent the air inlets 38 past the wick 30 and heating coil 28 to the air inhalation port 36.

In use, a user sucks on the mouthpiece portion 14 of the e-cigarette 10. This causes air to be drawn into the e-cigarette 10 via the air inlets 38 and to be drawn up via the central passage 32 towards the air inhalation port 36. The change in air pressure which arises is detected by the airflow sensor 24 which generates an electrical signal that is passed to the control electronics 22. In response to the signal, the control electronics 22 then proceed to activate the heating coil 28 which causes liquid present in the wick 30 to be vaporized creating an aerosol (which may comprise gaseous and liquid components) within the central passage 32. As the user continues to suck on the mouthpiece portion 14 of the e-cigarette 10, this aerosol is drawn up the central passage 32 and inhaled by the user sucking on the e-cigarette 10. At the same time the control electronics 22 also activate the LED 20 causing the LED 20 to light up which is visible via the translucent end cap 16 mimicking the appearance of a glowing ember at the end of a conventional cigarette. As liquid present in the wick 30 is converted into an aerosol more liquid is drawn into the wick 30 from the liquid store 34 by capillary action and thus is available to be converted into an aerosol through subsequent activation of the heating coil 28.

In some e-cigarettes, the e-cigarette 10 is intended to be disposable and the electric power in the battery 18 is intended to be sufficient to vaporize the liquid contained within the liquid store 34 after which the e-cigarette 10 is thrown away. In other embodiments the battery 18 is rechargeable and a means is provided to replenish the liquid supply. In the cases where the liquid store 34 is a toroidal cavity, this may be achieved by providing a refill port and refilling the cavity with liquid via the refill port. In other embodiments the mouthpiece portion 14 of the e-cigarette 10 is detachable from the main body 12 and a new mouthpiece portion 14 can be fitted with a new liquid store 34 thereby replenishing the supply of liquid. In some cases, replacing the liquid store 34 may involve replacement of the heating coil 28 and the wick 30 along with the replacement of the liquid store 34.

In some cases the new liquid store 34 may be in the form of a cartridge. In some such embodiments the cartridge may be provided with a central passage 32 through which a user inhales aerosol generated by the e-cigarette. In other embodiments, rather than inhaling aerosol via a central passage 32, the cartridge may be such to block the central portion of the e-cigarette 10 and generated aerosol may be directed around the exterior of the cartridge 32 to an air inhalation port 36 for inhalation.

It will also be appreciated that although the above description is illustrative of the structure and function of a typical e-cigarette 10, variations also exist. Thus for example in some e-cigarettes the LED 20 is omitted. In some e-cigarettes, the airflow sensor 24 may be placed adjacent the end cap 16 of the e-cigarette rather than in the middle of the e-cigarette as illustrated. Similarly, in some e-cigarettes, the air inlets 38 may be placed at the distal end of the main body 16 of the e-cigarette 10 remote from the mouthpiece portion 14. In some e-cigarettes the airflow sensor 24 is omitted and instead a button is provided which enables a user to activate the e-cigarette manually rather than in response to the detection of a change in air flow or air pressure. Also in some e-cigarettes the constitution of the atomizer may be changed. Thus for example rather than being constituted by a wick 30 surrounded by a heating coil 28 other configurations may be used such as providing a heating coil in a cavity in the interior of a porous body soaked in liquid for atomization and generating an aerosol by evaporating the liquid within the porous body either by virtue of the activation of the coil heating the porous body or alternatively by virtue of the heated air being passed over or through the porous body. Further in some embodiments rather than generating an aerosol through heating liquid within a wick 30 an aerosol might be generated using a piezoelectric atomizer to create an aerosol for inhalation either in combination or in the absence of a heater.

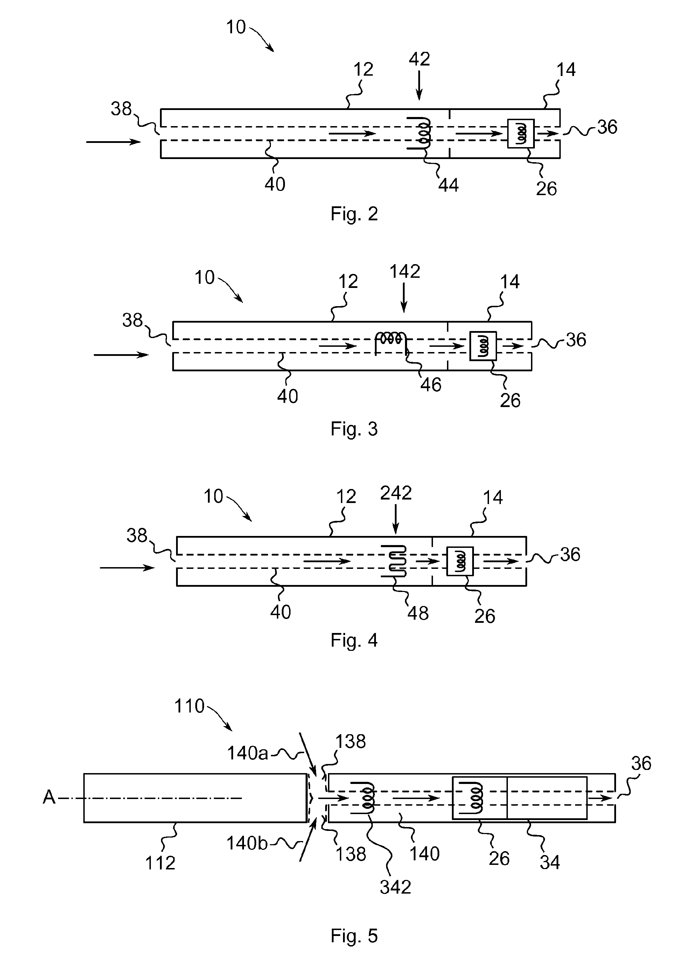

In Figure 2 an electronic smoking device in form of an e-cigarette 10 is depicted. In order to ease understanding of the present invention, some elements of the electronic cigarette 10 like for example the battery and the liquid store are omitted here. The battery is usually arranged inside the main body 12. The liquid store may be arranged here inside the main body 12. The atomizer 26 and its heating coil are depicted only in a schematic way.

In use, when a user puffs on the e-cigarette 10, ambient air enters the e-cigarette 10 through at least one air inlet 38. In the e-cigarette 10 of Figure 2, in contrast to the example of Figure 1, the air inlet 38 is provided at the distal end of the e-cigarette 10 remote from the mouthpiece. Inside the e-cigarette 10 the air proceeds along or through an air flow path 40. The flow path 40 connects the air inlet 38 and the air inhalation port 36. Arrows indicate the flow direction side the flow path 40.

The flow path 40 is defined by at least one of the side walls of the main body 12 and the mouthpiece portion 14 and inner parts of the e-cigarette 10 like for example the battery and the liquid store or the like. Part of the air flow path 40 or the complete air flow path 40 may be defined by a tube or a hose. In such cases, a tube or hose may connect two elements or sections of the air flow path 40. Elements in the air flow path 40 may encompass an air inlet or air inlets 38, the atomizer 26 or an atomizing chamber of the atomizer 26, the air inhalation port 36 and an optional air flow sensor 24.

In the example shown in Figure 2, the air flow path 40 progresses in a straight line from the air inlet 38 to the air inhalation port 36. In other embodiments, the air flow path 40 may comprise curved sections or may divide into two or more sub flow paths or partial flow paths.

An air pre-heating element 42 is arranged in the flow path 40 upstream of the atomizer 26. Here, the air pre-heating element 42 is arranged inside the main body 12 of the e-cigarette 10. Ambient air is heated by the air heating element 42 prior to being mixed with the aerosol created by the atomizer 26.

The applicants have appreciated that the performance of the atomizer 26 of an e-cigarette 10 can be adversely impacted if the ambient air temperature is low. Such issues are avoided or reduced if an air pre-heating element 42 is provided upstream of an atomizer 26 and the air pre-heating element 42 is used to heat the air in the flow path 40 before the air reaches the atomizer 26. Pre-heating the air in this manner stabilizes the performance of the e-cigarette across a wide range of different temperature environments. Further, the provision of heated air to an atomizer 26 may reduce condensation of droplets onto surfaces, increase vapor production, and give more consistent delivery than if atomized gas is mixed with air at a temperature was dependent on the ambient temperature, particularly when the ambient air temperature is relatively cold. In addition the heating of air by an air pre-heating element 42 in addition to heating arising due to the activation of an atomizer 26 may also provide warmer vape which is preferred by some consumers.

In Figure 2 the air pre-heating element 42 includes a coil 44 for heating the ambient air which is drawn by the consumer taking a puff. The coil 44 is arranged inside the air flow path 40 thereby heating the ambient air directly. Alternatively, the coil 44 may be arranged around the airflow path 40 thereby indirectly heating the air through a wall of the air flow path 40. Further, the air pre-heating element 42 may include an alternative heating element, such as a ceramic heater. The air pre-heating element 42 may further include baffles or fins in the air flow path to better transfer heat to the flowing air.

The windings of the coil 44 are wound around an axis arranged perpendicular to the flow path 40 without any physical body blocking the center of the coil 44. Such arrangement of the coil 44 allows for a good heat transfer to the passing air.

The coil 44 of the air pre-heating element 42 is connected with the battery of the e-cigarette 10. The air pre-heating element 42 may be activated by an optional air flow sensor which detects a puff of the consumer or when the consumer presses a button. Upon such activation a current flows from the battery through the windings of the coil 44 so that the coil 44 warms or heats up. Preferably, the coil 44 comprises heating wire.

Preferably, the air pre-heating element 42 and the atomizer 26 are two distinct units. Between these two units a relative distance of space is present. As air passes across such an arrangement, initially the air is heated by the air pre-heating element 42. Then, the heated air is transferred to the atomizer 26 where the liquid is vaporized and mixes with the heated air. The separation of the two units enables air heated by the air pre-heating element 42 to mix with any air which was not heated so that uniformly warm air is passed over the atomizer 26. Preferably, the air pre-heating element 42 and the atomizer 26 are separated by a distance which lies in the range of several millimeters to a few centimeters, and equals preferably to 10 millimeter. Further, the air pre-heating element 42 may be spaced apart from the liquid, so that it is not wet. In such fashion, the air pre-heating element 42 contacts only air and not the liquid from the liquid store.

Preferably, the heating temperature of the air pre-heating element 42 is lower than the heating or vaporizing temperature of the atomizer 26 or its heating coil 28. In this way the ambient air is warmed by the air pre-heating element 42 before being further heated by the activation of the atomizer 26. Having the air pre-heating element 42 warm air to a limited degree reduces power demands and hence extends battery life. A user will judge the performance of an e-cigarette 10 in terms of the vapor it produces. The level of heating by the air pre-heating element42 should therefore preferably be such to raise the temperature of resultant vapor to the level preferred by a user and to avoid or reduce re-condensation of atomized vapor within the e-cigarette 10. Limiting the heating by the air pre-heating element 42 avoids unnecessary heating of the e-cigarette 10 and limits the amount by which ambient air will be heated and then cool before reaching the atomizer 26. A difference in temperature between the respective temperatures of the air pre-heating element 42 and the vaporizing coil of the atomizer may exist. The vaporizing coil may always be hotter than the air pre-heating element 42. The vaporizing coil may reach temperatures between about 90° C and 250° C. The air pre-heating element 42 may reach temperatures between about 10° C and 70° C and preferably temperatures between about 20° C and 50° C.

Figure 3 shows an e-cigarette 10 having an air pre-heating element 142 similar to the one shown in Figure 2. The air pre-heating element 142 comprises a coil 46. Again, the coil 46 is electrically connected with the battery and is preferably made of heating wire or the like. The coil 46 has windings which are wound around an axis arranged in parallel to the flow path 40. The size of the coil 46, i.e. the diameter of the windings and/or the length in direction of the flow path or the number of windings, is chosen with regard to the physical dimensions of the air flow path 40 and/or a desired amount of heat which is to be transferred to the air in the air flow path 40 by the coil 46. The windings of the coil 46 may be arranged inside the air flow path 40 so that the coil 46 is inline with the flow of air through the air flow path 40. In such case, the air flows through the windings of the coil 46. Alternatively, the windings of the coil 46 may be arranged around the airflow path 40. In such case the coil 46 heats up the air indirectly as the air is drawn through the air flow path 40. Preferably, a wall or shell of the air flow path 40 comprises heat conducting material so as to improve heat transfer from the coil 46 to the air within the air flow path 40. Alternatively, the air pre-heating element 142 may be a heater in form of a pipe. Such pipe acts as a wall of the air flow path 40 and defines the air flow thereby directly heating the passing air.

Figure 4 shows a further embodiment of the e-cigarette 10 having an air pre-heating element 242 including a planar heating element 48. The planar heating element 48 extends in one plane. The normal vector of the plane in which the planar heating element 48 extends is parallel to the direction of flow. Alternatively, in other embodiments the planar heating element 48 could be arranged so that normal vector of the planar containing the planar heating element 48 is inclined at an angle relative to the direction of flow of air along the air flow path 40.

The planar heating element 48 may have the form of a mesh or a grill. Such arrangement may maximize the transfer of heat to the air. Preferably, the planar heating element 48 is arranged completely inside the air flow path 40. Only electrical connectors extend outside the air flow path 40.

Figure 5 shows a further embodiment of an e-cigarette 110. The e-cigarette 110 in this embodiment includes a main body 112 in which a battery is located and a mouthpiece portion 114. The atomizer 26 and the liquid store 34 are arranged inside the mouthpiece portion 114. In such an embodiment, the mouthpiece portion 114, i.e. a unit including a cartridge or a liquid store and the atomizer can be designated as a cartomizer. In Figure 5 the atomizer 26, the liquid store 34 and an air pre-heating element 342 are arranged inside the mouthpiece portion 114. Accordingly, only an electrical connection needs to be provided between the main body 112 and the mouthpiece portion 114 for supplying electrical energy from the battery located inside the main body 112 to the air pre-heating element 342 and the atomizer 26 located inside the mouthpiece portion 114.

In the transition between the main body 112 and the mouthpiece portion 114 air inlets 138 are provided. Alternatively, the air inlets 138 are provided at a second end of the mouthpiece portion 114 being opposed to a first end including the air inhalation port 36.

An air flow path 140 extends from the air inlets 138 to the air inhalation port 36. In between the air flow path 140 passes the air pre-heating element 342 and the atomizer 26. At the second end of the mouthpiece portion 114 the air flow path 140 includes two partial flow paths 140a and 140b. Each air inlet 138 defines a beginning of the partial flow paths 140a, 140b. The two partial flow paths 140a, 140b meet or join upstream of the air pre-heating element 342 so that the air pre-heating element 342 is associated with both of the two partial flow paths 140a, 140b. As an alternative, only one of the partial flow paths is associated with the air pre-heating element 342. The other flow path then bypasses the air pre-heating element 342 and directly continues to the atomizer 26. Such an arrangement may have benefits in mixing the different air streams inside the atomizer 26.

The heater of the air pre-heating element 342 may be implemented as shown in Figures 2 to 4. While according to Figures 2 to 4 only one air inlet 38 is provided at the distal end of the e-cigarette 10 Figure 5 illustrates two or more air inlets 138 which are arranged laterally. Combination of both arrangements are possible.

The air pre-heating element 342 is arranged adjacent to the atomizer 26 in a direction of a longitudinal main axis A of the electronic smoking device 110. The term adjacent includes the cases of being directly adjacent, i.e. in contact with each other, and the case of neighboring, i.e. being located at a distance to each other. The arrangement along the main axis A of the electronic smoking device 110 may have the advantage of evenly distributing the inner parts of the e-cigarette 110 and thereby balancing out the e-cigarette 110.

Alternatively, the air pre-heating element 342 may be arranged adjacent to the atomizer in a direction perpendicular to a longitudinal main axis A of the electronic smoking device 110. Such arrangement may allow decreasing the length of the mouthpiece portion 114 which may ease the handling of the e-cigarette 110.

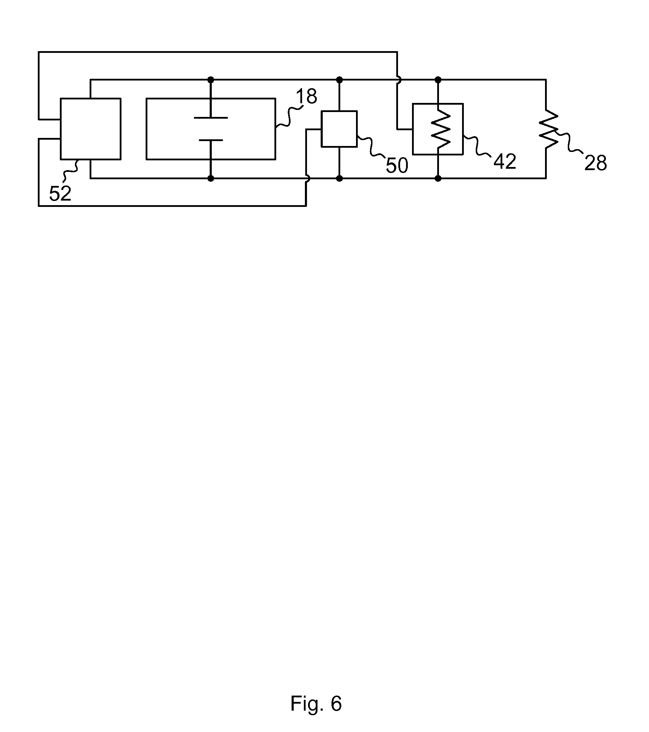

Figure 6 shows a schematic wiring diagram of an e-cigarette 10. Terminals of the battery 18 are connected with corresponding terminals of the air pre-heating element 42. The heating coil 28 of the atomizer 26 is electrically connected in parallel to the air pre-heating element 42. The electrical resistances of the heating coil 28 and the air pre-heating element 42 do not have to be equal. One or more switches may be included to selectively enable at least one of the heating coil 28 and the air pre-heating element 42. The one or more switches may be controlled by an optional puff sensor or a consumer activated button.

In an optional design a temperature sensor 50 may be provided to measure the temperature of the ambient air. The temperature sensor 50 may be located between the air inlet 38 or 138 and the air pre-heating element 42. Alternatively, the temperature sensor 50 may be located at the outside or at an end of the main body 12 or the mouthpiece portion 14. The temperature sensor 50 is also connected with the battery 18. The temperature sensor 50 may be active all the time or only when the air pre-heating element 42 is activated. In addition or as an alternative to the temperature sensor 50 an air flow sensor may be provided for sensing air flow velocity of the ambient air flowing at the air pre-heating element 42 or into the atomizer. Power requirements for the air pre-heating element 42 may then vary with the detected air flow velocity. In detail, the current to the air pre-heating element 42 may be adjusted based on the detected air flow velocity. For a higher air flow velocity the current may be increased while the current may be decreased for a lower air flow velocity. Such design may maintain a near constant air temperature for air flowing into the atomizer.

Further, a control unit 52 may be provided. The control unit 52 is powered by the battery 18. As an input, the control unit 52 receives an output of the temperature sensor 50. The control unit 52 may further receive an input from an optional puff sensor or a consumer activated button in order to activate at least one of the air pre-heating element 42 and the heating coil 28.

The control unit 52 is further connected with the air pre-heating element 42. In accordance with the measured temperature the control unit 52 controls the air pre-heating element 42 such that a desired temperature of the ambient air is achieved. It may be of advantage that the control unit 52 heats the ambient air to a predetermined temperature or temperature range irrespective of the ambient temperature. Based on the temperature of the ambient air the control unit 52 determines how much heat has to be transferred to the ambient air by the air pre-heating element 42. Such arrangement may have the advantage of providing precisely temperate air to the atomizer 42 thereby employing perfect conditions for the heating coil 28.

The schematic wiring diagram of Figure 6 corresponds to the structure any of the e-cigarettes 10 shown in Figures 2-5 subject to the replacement of the reference number for the air pre-heating element 42 of Figure 2 being replaced with a reference to the air pre-heating elements 142, 242 and 342 of Figures 3 to 5.

While this invention has been described in connection with what is presently considered to be practical exemplary embodiments, it is to be understood that the invention is not limited to the disclosed embodiments, but, on the contrary, is intended to cover various modifications and equivalent arrangements included within the scope of the appended claims.

### LIST OF REFERENCE SIGNS

- 10, 110: e-cigarette
- 12, 112: main body
- 14, 114: mouthpiece portion
- 16: end cap
- 18: battery
- 20: light emitting diode (LED)
- 22: control electronics
- 24: airflow sensor
- 26: atomizer
- 28: heating coil
- 30: wick
- 32: central passage
- 34: liquid store
- 36: air inhalation port
- 38, 138: air inlets
- 40, 140: air flow path
- 42, 142, 242, 342: air pre-heating element
- 44: coil
- 46: coil
- 48: planar heating element
- 50: sensor
- 52: control unit
- 140a: partial flow path
- 140b: partial flow path
- A: main axis

## Claims

1. Cartomizer adapted for an electronic smoking device (10), comprising:
- an elongated, hollow body (14) having a mouthpiece opening (36) in a first end and a coupling adapted to couple to a main body (12) of an electronic smoking device (10) in an opposite second end;
- an atomizer (26) operable to create an aerosol for inhalation by a user through the mouthpiece opening (36) by atomizing a liquid supplied from a liquid store (34) and arranged inside the body (14), wherein an air flow path (40) extends from an air inlet (38) to the atomizer (26); and
- an air pre-heating element (42) arranged in the flow path (40) upstream of the atomizer (26).

2. Electronic smoking device, comprising:
- a main body (12) including an electric power source (18);
- a mouthpiece portion (14) having a mouthpiece opening (36) in an end;
- at least one air inlet (38) arranged in at least one of the main body (12) and the mouthpiece portion (14);
- an atomizer (26) arranged in the mouthpiece portion (14) and operable to create an aerosol for inhalation by a user through the mouthpiece opening (36), wherein an air flow path (40) extends from the at least one air inlet (38) to the atomizer (26), wherein the atomizer (26) is connected with the electric power source (18) for powering the atomizer (26); and
- an air pre-heating element (42) arranged in the flow path (40) upstream of the atomizer (26).

3. Electronic smoking device of any of the previous claims, wherein the air pre-heating element (42) comprises a coil.

4. Electronic smoking device of claim 3, wherein the air pre-heating element (142) comprises a coil (46) having windings wound around an axis arranged in parallel to the air flow path (40).

5. Electronic smoking device of claim 3, wherein the air pre-heating element (42) comprises a coil (44) having windings wound around an axis arranged perpendicular to the air flow path (40).

6. Electronic smoking device of claim 1 or claim 2, wherein the air pre-heating element (242) comprises a planar heating element (48).

7. Electronic smoking device of claim 1 or claim 2, wherein the air pre-heating element comprises a heater in form of a pipe.

8. Electronic smoking device of any of the previous claims, further comprising a temperature sensor (50) adapted to measure the temperature of the ambient air and a control unit (52) for controlling the air pre-heating element (42) in accordance with the measured temperature.

9. Electronic smoking device of any of the previous claims, wherein the air pre-heating element (42) is electrically connected in parallel with a heating coil (28) of the atomizer (26).

10. Electronic smoking device of any of the previous claims, comprising at least two air inlets (138) each defining a beginning of at least two partial flow paths (140a, 140b), wherein the air pre-heating element (138) is associated with at least one of the at least two partial flow paths (140a, 140b).

11. Electronic smoking device of any of the previous claims, wherein the air pre-heating element (42) is arranged adjacent to the atomizer (26) in a direction of a longitudinal main axis (A) of the electronic smoking device.

12. Electronic smoking device of any of the previous claims, wherein the air pre-heating element is arranged adjacent to the atomizer (26) in a direction perpendicular to a longitudinal main axis (A) of the electronic smoking device.

13. Method generating a vapour for inhalation using an electronic smoking device (10) comprising at least one air inlet (38) and an atomizer (26), wherein the method comprises:
drawing ambient air into the electronic smoking device (10) via the at least one air inlet (38);
heating air within an air flow path (40) extending between the at least one air inlet (38) and the atomizer (38) utilising an air pre-heating element (42);
providing heated air to the atomiser (26); and
mixing the heated air with atomised vapour generated by the atomizer (26).

14. The method of claim 13, wherein the activation of the air pre-heating element (42) is initiated by the detection of an air flow within the airflow path (40).

15. The method of claim 13 or 14, further comprising:
detecting the ambient air temperature; and
inhibiting the activation of the air pre-heating element (42) if the ambient air temperature is greater than a predetermined threshold temperature.
